# EUROPÄISCHE PATENTANMELDUNG

(11) **EP 1 752 139 A1**
(43) Veröffentlichungstag der Anmeldung: **14.02.2007**
(21) Anmeldenummer: 06014542.2
(22) Anmeldetag: 13.07.2006
(51) Int. Cl.: A61K 9/00, A61K 31/167

(54) **Stabile, flüssige Formulierung von Paracetamol**

(30) Priorität: 05.08.2005 DE 102005037653
(71) Anmelder: TheraSelect GmbH, 80336 München (DE)
(72) Erfinder: Guardiola, Jaime, 66242 Homburg (DE)
(74) Vertreter: Binsack, Beate

(57) **Zusammenfassung**

Die Erfindung betrifft eine stabile, flüssige Formulierung von Paracetamol in einem wässerigen Medium enthaltend
a) etwa 1 bis etwa 17 g/l Paracetamol und
b) etwa 0,01 bis etwa 0,4 g/l eines oder mehrerer physiologisch verträglicher Antioxidantien ausgewählt aus der Gruppe bestehend aus Ascorbinsäure, N-Acetyl-L-cystein, und SH-Gruppenhaltiger Stabilisator-Verbindungen, die von N-Acetyl-L-cystein verschieden sind,

wobei die Formulierung im wesentlichen frei von organischen Lösungsmitteln ist, kein Puffersystem enthält und einen Sauerstoffgehalt von weniger als etwa 1 mg/l aufweist.

## Beschreibung

Die Erfindung betrifft eine stabile, flüssige Formulierung von Paracctamol in einem wässerigen Medium sowie deren Verwendung als infundierbares Analgetikum.

Ein seit Jahrzehnten etabliertes Standardanalgetikum ist Paracetamol. Dieses weist sehr günstige pharmakologische Eigenschaften auf: analgetische und antipyretische Wirkung bei sehr geringer antiphlogistischer Wirkung. Daher wird es beispielsweise routinemäßig zur postoperativen Schmerztherapie bereits im Kindesalter eingesetzt. Der Einsatz wird dabei vorwiegend prophylaktisch bei der Narkoseeinleitung oder während des operativen Eingriffs vorgenommen und erfolgt meistens rektal.

Als intravenöses Arzneimittel ist Paracetamol als steriles Pulver in Form eines Ester-Prodrugs im Handel (Pro-Dafalgam, Wirkstoff Paracetamol, chemisch der Diethylaminoglycinester des Paracetamols). Diese Arzneiform muss zunächst rekonstituiert und dann mit einer Infusions-Trägerlösung, wie z.B. Glucose 5 % oder isotonischer Kochsalzlösung, auf eine geeignete Fertiginfusionslösung weiter verdünnt werden. Dieser Vorgang ist aufwändig. Außerdem ist die Herstellung des Prodrugs und die Gefriertrocknung des Esters zum sterilen Lyophilisat mit hohen Herstellungskosten verbunden.

In der EP 0 916 347 A1 sind Versuche zur Herstellung einer fertigen

Paracetamol-Injektionsform beschrieben, bei denen mit Hilfe von organischen Lösungsmitteln eine genügende Löslichkeit des Paracetamols erreicht wird, so dass für intramuskuläre Injektionsformen, bei denen das Dosiervolumen natürlicherweise limitiert ist, in kleinen Volumina ausreichende Mengen des Paracetamols gelöst werden können. Die EP 0 916 347 A1 offenbart dabei für die Lösung in organischen Lösungsmitteln weiterhin einen optimalen pH-Wert von 5,5 bis 5,6 sowie den Einsatz von Stabilisatoren wie Natrium-EDTA und Natrium-Bisulfit.

Letzteres Antioxidans hat bekanntlich gravierende Nachteile in Form von unerwünschten Nebenwirkungen, die seinen Einsatz inzwischen obsolet machen.

Für Infusionslösungen des Paracetamols, bei denen höhere Volumina intravenös gegeben werden, ist eine Löslichkeit des Wirkstoffes in dem Maße, wie sie in der EP 0 916 347 A1 angegeben wird, nicht essentiell. Zum Beispiel wird Pro-Dafalgam nach Rekonstitution in 20 ml Citratpuffer mit 100 ml isotoner Kochsalzlösung bzw.5 %iger Glueoselösung weiter zur applikationsfertigen Infusionslösung verdünnt. Für eine solche Präparation wäre an sich die Wasserlöslichkeit des Paracetamols ausreichend. Dennoch sind im Handel Fertiginfusionslösungen des Paracetamols aufgrund von Stabilitätsproblemen so gut wie nicht anzutreffen.

Es ist bekannt, dass wässerige Lösungen des Paracetamols schon nach geringen Lagerzeiten zur Ausbildung von Verfärbungen neigen. Darüber hinaus treten Trübungen auf und es werden Partikel bzw. Niederschläge gebildet. Entsprechend gealterte Infusionslösungen des Paracetamols können selbstverständlich nicht mehr verwendet werden.

Die Instabilität des Paracetamols in wässeriger Lösung ist auf eine Verseifung des hydrolyseempfindlichen Amids Paracetamol zurückzuführen. Sie ist im Stand der Technik als eine Funktion des pH-Wertes der Lösung ausführlich beschrieben (z.B. K. T. Koshy, J. I. J. Lach, Pharm. Sci., 50 (1961), 113-118). Daher wird im Stand der Technik der pH-Wert eingestellt, bei dem das Paracetamol am stabilsten ist und dieser pH-Wert wird mittels eines Puffersystems konstant gehalten.

Die EP 858 329 B1 beschreibt eine flüssige, gegen Oxidation stabile Rezeptur auf der Basis von Paracetamol in einem wässerigen Lösungsmittel, bei der das Paracetamol in der wässerigen Lösung mit Hilfe einer zugegebenen Pufferlösung stabilisiert wird.

Die WO 02/072080 offenbart lagerstabile Fertiginfusionslösungen des Paracetamols, die einen pH-Wert im Bereich von 5,5 bis 6,5 aufweisen. Dieser wird mit Hilfe eines Puffersystems, bevorzugt eines Phosphatpuffers, eingestellt.

Die WO 20041071502 A1 beschreibt eine injektierbare flüssige Formulierung von Paracetamol, die ein wässeriges Lösungsmittel, ein Pufferagens mit einem pKa-Wert zwischen 4,5 und 6,5, ein isotonisches Agens und das Dimere des Paracetamols enthält.

Aufgabe der vorliegenden Erfindung ist es, die Nachteile des Standes der Technik zu überwinden und eine lagerstabile, physiologisch akzeptable und kostengünstig herstellbare Paracetamol-Infusionslösung zur Verfügung zu stellen.

Diese Aufgabe wird erfindungsgemäß durch die kennzeichnenden Merkmale des Hauptanspruchs und des nebengeordneten Anspruchs gelöst. Bevorzugte Ausführungsformen der Erfindung sind in den Unteransprüchen und der nachfolgenden Beschreibung definiert.

Es war überraschend und für den Fachmann in keiner Weise vorhersehbar, dass die Verwendung einer pufferfreien Paracetamol-Lösung mit einem Restsauerstoff-Gehalt von weniger als etwa 1 mg/l und dem Einsatz eines Antioxidanten den Nachteilen des Standes der Technik abhelfen würde und zu einer lagerstabilen Formulierung führt, bei der kaum ein Abbau des Wirkstoffes stattfindet. Überraschenderweise gelang es also auch ohne die Verwendung eines Pufferagens eine stabile wässerige Paracetamol-Lösung zur Verfügung zu stellen, d.h., die Formulierung weist auch nach längerer Lagerung keine Verfärbungen auf und entwickelt bei der Lagerung keine Partikel oder Niederschläge.

Dies ist um so überraschender, da dem Fachmann aus dem Stand der Technik nur bekannt ist, dass Paracetamol ein hydrolyseempfindliches Amid ist und dass er für hydrolyseempfindliche Substanzen wie Amide ein Puffersystem verwenden sollte.

Ein erster Gegenstand der vorliegenden Erfindung ist daher eine stabile, flüssige Formulierung von Paracetamol in einem wässerigen Medium enthaltend
a) etwa 1 bis etwa 17 g/l Paracetamol und
b) etwa 0,01 bis etwa 0,4 g/l eines oder mehrerer physiologisch verträglicher Antioxidantien ausgewählt aus der Gruppe bestehend aus Ascorbinsäure, N-Acetyl-L-cystein, und SH-Gruppen-haltiger Stabilisator-Verbindungen, die von N-Acctyl-L-cystein verschiden sind,
wobei die Formulierung im wesentlichen frei von organischen Lösungsmitteln ist, kein Puffersystem enthält und einen Sauerstoffgehalt von weniger als etwa 1 mg/l aufweist.

Diese erfindungsgemäße wässerige Formulierung des Paracetamols ist parenteral verabreichbar, insbesondere infundierbar.

Die Formulierung von Paracetamol gemäß der Erfindung enthält erfindungsgemäß kein Puffersystem. Auch wenn der pH-Wert eine entscheidenden Einfluss auf die Stabilität dieser wässerigen Lösung haben kann, kann er erfahrungsgemäß über einen gewissen Bereich variieren. Er darf sich jedoch - speziell bei Injektabilia und Infundabilia - nicht deutlich von dem entsprechenden Wert im Blut unterscheiden, da sonst die Gefahr von Unverträglichkeiten auftritt. Der pH-Wert der erfindungsgemäßen Formulierung wird durch die Rezeptur festgelegt und wird - falls notwendig - im Rahmen der In-Prozesskontrolle beim Ansetzen der erfindungsgemäßen Formulierung beispielsweise mit Salzsäure und/oder Natronlauge eingestellt. In einer bevorzugten Ausführungsform liegt der pH-Wett zwischen etwa 4 und etwa 7 und ganz besonders bevorzugt zwischen etwa 5,5 und etwa 6,5.

Ein essentieller Bestandteil der erfindungsgemäßen Formulierung ist das Paracetamol. Die Menge des in der Formulierung gemäß der Erfindung enthaltenen Paracetamols kann über einen gewissen Bereich variieren. Angestrebt wird je nach Einsatzzweck eine möglichst hohe Konzentration an Paracetamol in der Lösung. Die Löslichkeit des Paracetamols in Wasser bei Raumtemperatur beträgt etwa 17 g/l. Die Löslichkeit kann jedoch in Abhängigkeit von in der Lösung enthaltenen weiteren Zusätzen auch höher als etwa 17 g/l sein. Für eine Reihe von Applikationen ist es zweckmäßig, dass die erfindungsgemäße Formulierung etwa 5 bis etwa 15 g/l, vorzugsweise etwa 7,5 bis etwa 12,5 g/l, an Paracetamol aufweist. Eine besonders applikationsfreundliche und für die meisten Standardfälle hervorragend geeignete Lösung weist einen Gehalt an Paracetamol-Wirkstoff von etwa 10 g/l auf.

Die Formulierung von Paracetamol gemäß der Erfindung enthält als weiteren essentiellen Bestandteil ein physiologisch verträgliches Antioxidationsmittel. Hierbei handelt es sich um Verbindungen, die aus der Gruppe ausgewählt sind, die aus Ascorbinsäure, N-Acetyl-L-cystein und SH-Gruppen-haltigen Stabilisator-Verbindungen, die vom N-Acetyl-L-cystein verschieden sind, besteht. Zu letzterer Gruppe gehört z. B. Cystein. Durch die Verwendung dieser Substanzen ist vorteilhafterweise eine besonders wirksame Stabilisierung auch über längere Lagerzeiten möglich ist, ohne dass die Verträglichkeit der Formulierung des Paractamols darunter leidet. Besonders bevorzugt unter den für die Erfindung in Frage kommenden antioxidativ wirkenden Verbindungen ist N-Acetyl-L-cystein. Selbstverständlich kann man als Stabilisator eine einzige Verbindung einsetzen. Es können aber auch Mischungen aus zwei oder mehr der stabilisierenden Verbindungen zur Anwendung gelangen.

Die Konzentration der stabilisierenden Verbindungen in der erfindungsgemäßen wässerigen Formulierung liegt im Bereich zwischen etwa 0,01 und etwa 0,4 g/l. In bevorzugter Ausführungsform weist die Formulierung etwa 0,05 bis etwa 0,2 g/l, vorzugsweise etwa 0,075 bis etwa 0,15 g/l der stabilisierenden Verbindung auf. Insbesondere bevorzugt als stabilisierende Verbindung wird N-Acetyl-L-cystein verwendet.

Das Verhältnis von Paracetamol a) zu stabilisierender Verbindung b) liegt bezogen auf das Gewicht der Verbindungen vorteilhafterweise im Bereich von etwa 10:1 bis etwa 1000:1. Besonders bevorzugt ist ein Gewichtsverhältnis von a) zu b) im Bereich von etwa 50:1 bis etwa 500:1, ganz besonders bevorzugt von etwa 80:1 bis etwa 200:1. Sehr gute Stabilitätsdaten werden dann erzielt, wenn das Gewichtsverhältnis von a) zu b) etwa 100:1 ist.

Die erfindungsgemäße Lösung ist in Wesentlichen frei von organischen Lösungsmitteln. Hierunter fallen alle dem Fachmann bekannten, im wesentlichen niedermolekularen organischen Verbindungen, die ausschließlich zum Zwecke der Erhöhung der Löslichkeit des Paracetamols in Wasser eingesetzt werden. Hierzu gehören vor allem Alkohole wie Ethanol Benzylalkohol und andere niedermolekulare Verbindungen, die Hydroxygruppen enthalten.

Die Stabilität der erfindungsgemäßen Formulierung wird durch eine Reduktion des Gehaltes an freiem Sauerstoff in der Lösung gesteigert. Erfindungsgemäß beträgt der Sauerstoffgehalt der Lösung weniger als etwa 1 mg/l. Bevorzugt ist der Sauerstoffgehalt jedoch so gering wie möglich zu halten. Von besonderem Interesse sind hierbei Lösungen, die sich durch einen Sauerstoffgehalt von weniger als etwa 0,50 mg/l, besonders bevorzugt von weniger als etwa 0,25 mg/l, und ganz besonders bevorzugt von weniger als etwa 0,10 mg/l, auszeichnen.

Insbesondere die Kombination von speziellen Antioxidantien und dem niedrigen Sauerstoffgehalt der Formulierung führt zu besonders lagerstabilen Formulierungen.

Die Formulierung, wie sie Gegenstand der Erfindung ist, kann weitere Zusätze aufweisen. So kann es sinnvoll sein, die Paracetamol-Lösung zu isotonisieren. In einer bevorzugten Ausführungsform enthält die erfindungsgemäße Formulierung daher ein oder mehrere Isotonisierungsmittel, vorzugsweise NaCl und/oder Glycerin. Unter physiologischen Aspekten ist der Einsatz von Natriumchlorid besonders geeignet, während die Verwendung von Glycerin die Löslichkeit des Paracetamols verbessern kann.

Die erfindüugsgemäße Formulierung kann zusätzlich zum Paracetamoi einen oder mehrere weitere pharmazeutische Wirkstoffe enthalten. Von besonderem Interesse für bestimmte Anwendungen sind hierbei Wirkstoffe aus der Gruppe der Lokalanästhetika, Analgetika und Spasmolytika.

Die Herstellung einer erfindungsgemäßen Formulierung bereitet dem Fachmann keinerlei Schwierigkeiten. Sie erfolgt beispielsweise, indem
a) Wasser für Injektionszwecke mit einem Sauerstoffgehalt von weniger als 0,50 mg/l vorgelegt wird und
b) anschließend Paracetamol sowie die weiteren Zusätze, wie beispielsweise Antioxidantien, Isotonisierungsmittel und gegebenenfalls weitere pharmazeutische Wirkstoffe in den gewünschten Mengen unter möglichst weitgehendem Ausschluss von Sauerstoff in der Vorlage gelöst werden.

Zweckmäßigerweise wird die Formulierung dann
c) durch ein Membranfilter 0,2 m filtriert und abschließend in Behälter für Infusionslösungen abgefüllt und sterilisiert.

Die Sterilisation der erfindungsgemäßen Formulierung bereitet dem Fachmann keinerlei Schwierigkeiten. Sie erfolgt in der Regel - wie bei sterilen Arzneimittelformen üblich - im Endbehältnis (mittels Autoklaven). Die Standard-Sterilisationsbcdingungen sind im Arzneibuch beschrieben (mindestens 15 Minuten bei > 121 °C). Es sind aber auch so genannten Alternativverfahren möglich (weniger Hitzebelastung, mildere Autoklavierungsbedingungen) vorausgesetzt der Anwender beweist die gleiche Sterilitätssicherheit verglichen mit dem Standard-Sterilisationsverfahren. Dies wird des öfteren bei temperaturlabilen Wirkstoffen abgewendet und ist auch für die erfindungsgemäßen Formulierungen vorteilhaft. Erfindungsgemäß bevorzugte Sterilisationsbedingungen lauten daher: Letalitätswert Fo ≥ 8 bei 121 °C.

Die erfindungsgemäße wässerige Formulierung von Paracetamol eignet sich sehr gut, um intravenös gegeben zu werden. Eine Rekonstitution ist dabei aufgrund der Lagerstabilität der erfindungsgemäßen Formulierung nicht notwendig.

Ein weiterer Gegenstand der vorliegenden Erfindung betrifft daher die Verwendung einer erfindungsgemäßen wässerigen Formulierung von Paracetamol als applikationsfertige Infusionslösung sowie deren Verwendung als infundierbares Analgetikum.

Die vollständige Offenbarung aller vor- und nachstehend aufgeführten Anmeldungen, Patente und Veröffentlichungen sind durch Bezugnahme in diese Anmeldung eingeführt.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

### Beispiele

### Beispiel 1: Herstellung einer Paracetamol-Infusionslösung

1. 175-180 l Ansatzwasser werden vorgelegt und auf 50-55°C erwärmt (Endvolumen 200 1).
2. Zugabe von Natriumchlorid (9 g/l bzw. 1,8 kg/200 1)
3. Stickstoffbegasung (N 4,6) und Rührer einschalten. Evakuieren des Ansatzes während des Begasens.
4. Nach vollständigem Lösen der Bestandteile wird die Lösung auf mindestens 30°C herunter gekühlt.
5. Die Sauerstoffkonzentration in der Lösung wird gemessen (maximal 0,2 mg/l).
6. Bei einem Restsaucrstoffgehalt von ≤ 0,2 mg/l und einer Temperatur von ≤ 30 °C werden die restlichen beiden Komponenten zugegeben:
   - Acetylcystein (100 mg/l bzw. 20 g/200 1)
   - Paracetamol (10 g/l bzw. 2 kg/200 1)
   Die Stickstoffbegasung wird erst nach dem vollständigen Lösen der Komponenten abgestellt. In-Prozesskontrolle Ansatz (Dichte, pH-Wert und Extinktion bei 420 nm). Dann wird auf das Endvolumen bei 20 bis maximal 25 °C aufgefüllt.
7. Die pH-Wert-Einstellung (6,0 ± 0,1) erfolgt gegebenenfalls mit HCl oder NaOH.
8. Die Begasung wird von Stickstoff auf Argon 4,8 umgestellt. Die Lösung wird unter Argon-Überdruck aufbewahrt.
9. Es folgt die Sterilisation der Produktleitung und des Filters.
10. Abkühlung der Leitung und Einblasen von Argon
   a) auf der unreinen Seite des Filters über den Teststutzen
   b) nach dem Filter über einen Stutzen und
   c) über die Entlüftung der Produktleitung zum Füller.
11. Das Ende der Produktleitung wird mit dem Ansatzkessel verbunden und es wird im Kreislauf unter Argonbegasung umgepumpt.
   Der Sollwert der Restsauerstoffkontrolle beträgt 0,5-0,7 mg/l.
12. Abfüllung erfolgt in 100 ml Infusionsflaschen der Behältnisklasse 2, Gummistopfen FM 257 BrBu/Helvoci
   Maximales Vakuum einstellen, Musterflaschen kräftig schütteln, 10 Minuten stehen lassen und den Sauerstoffgehalt in der Lösung erneut messen (Sollwert: 0,5-0,7 mg/l). Zweite In-Prozesskontrolle.
13. Sterilisation 121 °C (-1/-0)/Fo>8<10.

### Beispiel 2: Herstellung einer Paracetamol-Infusionslösunp, (Variante:Menge Acetylcystein (Herstellungsschritt 6))

6. Bei einem Restsauerstoffgehalt von ≤ 0,2 mg/l und einer Temperatur von ≤ 30 °C werden die restlichen beiden Komponenten zugegeben:
   - Acetylcystein (300 mg/l bzw. 60 g/200 1)
   - Paracetamol (10 g/l bzw. 2 kg/200 1)

### Beispiel 3: Herstellung einer Paracetamol-Infusionslösung (Variante:Verwendung eines Gummistopfen Herstellungsschritt 12))

12. Abfüllung erfolgt in 100 ml Infusionsflaschen der Behältnisklasse 2 mit Gummistopfen aus Chlorbutyl gem. Ph. Eur.

## Patentansprüche

1. Stabile, flüssige Formulierung von Paracetamol in einem wässerigen Medium enthaltend
a) etwa 1 bis etwa 17 g/l Paracetamol und
b) etwa 0,01 bis etwa 0,4 g/l eines oder mehrerer physiologisch verträglicher Antioxidantien ausgewählt aus der Gruppe bestehend aus Ascorbinsäure, N-Acetyl-L-cystein, und SH-Gruppen-haltiger Stabilisator-Verbindungen, die von N-Acetyl-L-cystein verschieden sind,
wobei die Formulierung im wesentlichen frei von organischen Lösungsmitteln ist, kein Puffersystem enthält und einen Sauerstoffgehalt von weniger als etwa 1 mg/l aufweist.

2. Formulierung nach Anspruch 1, **dadurch gekennzeichnet, dass** der pH-Wert der wässerigen Formulierung im Bereich von etwa 4 bis etwa 7, vorzugsweise zwischen etwa 5,5 und etwa 6,5, liegt.

3. Formulierung nach Anspruch 1 oder 2, **dadurch gekennzeichnet, dass** sie etwa 5 bis etwa 15 g/l, vorzugsweise etwa 7,5 bis etwa 12,5 g/l Paracetamol enthält.

4. Formulierung nach einem der Ansprüche 1 bis 3, **dadurch gekennzeichnet, dass** sie etwa 0,05 bis etwa 0,2 g/l, vorzugsweise etwa 0,075 bis etwa 0,15 g/l N-Acetyl-L-cystein enthält.

5. Formulierung nach einem der Ansprüche 1 bis 4, **dadurch gekennzeichnet, dass** sie einen Sauerstoffgehalt von weniger als etwa 0,50 mg/l, vorzugsweise weniger als etwa 0,25 mg/l, aufweist.

6. Formulierung nach einem der Ansprüche 1 bis 5, **dadurch gekennzeichnet, dass** sie ein oder mehrere Isotonisierungsmittel, vorzugsweise NaCl und/oder Glycerin, enthält.

7. Formulierung nach einem der Ansprüche 1 bis 6, **dadurch gekennzeichnet, dass** sie in Mischung mit Paracetamol einen oder mehrere weitere pharmazeutische Wirkstoffe aus der Gruppe der Lokalanästlietika, Analgetika und Spasmolytika enthält.

8. Verwendung einer wässerigen Formulierung von Paracetamol nach einem der Ansprüche 1 bis 7 als Fertiginfusionslösung.

9. Verwendung einer wässerigen Formulierung von Paracetamol nach einem der Ansprüche 1 bis 7 als infundierbares Analgetikum.
